Europäisches Patentamt

European Patent Office

Office européen des brevets

(11) Publication number : **0 471 566 A1**

# (12) EUROPEAN PATENT APPLICATION

(21) Application number : **91307505.7**

(22) Date of filing : **14.08.91**

(51) Int. Cl.⁵ : **C07C 51/363,** C07C 57/13

(30) Priority : **17.08.90 GB 9018144**

(43) Date of publication of application :
**19.02.92 Bulletin 92/08**

(84) Designated Contracting States :
**AT BE CH DE DK ES FR GB GR IT LI NL SE**

(71) Applicant : **UNICHEMA CHEMIE BV**
**Buurtje 1**
**NL-2802 BE Gouda (NL)**

(72) Inventor : **Cross, Graham Andrew**
**Ramlehweg 28a**
**NL-3061 JZ Rotterdam (NL)**
Inventor : **Haase, Klaus Dieter**
**Van der Heimstraat 4**
**NL-2582 RZ Den Haag (NL)**
Inventor : **Laane, Nicolaas Charles Marie**
**A. van der Doeslaan 20**
**NL-3054 ED Rotterdam (NL)**
Inventor : **Vreeswijk, Johannes Josephus**
**Stekelbrem 62**
**NL-3068 TD Rotterdam (NL)**

(74) Representative : **Gambell, Derek et al**
**UNILEVER PLC Patents Division P.O. Box 68**
**Unilever House**
**London EC4P 4BQ (GB)**

(54) **Polymerisation process.**

㊼   A process is described for the polymerisation of unsaturated fatty acids, especially containing oleic acid, whereby the acid is heated to 175°C in the presence of a catalyst for a period sufficient for less than 40% of the acid to be polymerised. The product dimer acid is easier to hydrogenate than if polymerisation is continued further. The remaining unpolymerised material undergoes cis-trans isomerisation.

EP 0 471 566 A1

Jouve, 18, rue Saint-Denis, 75001 PARIS

This invention relates to a process for the polymerisation of unsaturated fatty acids, wherein the fatty acid is heated to an elevated temperature in the presence of a polymerisation catalyst.

The main products of such a process are dimers which know many applications dependent on their physical and chemical properties. Branched fatty acids are also formed in the reaction, generically referred to as "isostearic acid".

Such processes are known in the art. In US 3632822 (Conroy/Arizona Chemical Company) a process is described in which mixtures of unsaturated higher fatty acids consisting predominantly of oleic and (non-conjugated) linoleic acids are polymerised in two stages. The first stage takes place in the presence of an alkaline earth metal salt modified mineral clay until about 40% to about 60% of the acids have polymerised. Residual unpolymerised acids are separated and dimerised in a second stage in the presence of an acid-activated clay.

In British patent application specification GB 2172597A (Union Camp Corporation) a process is described for polymerising unsaturated fatty acids by heating in the presence of unactivated clay until the yield of the polymer based on the fatty acid mixture is on the order of 40-60%, after which the product is distilled to separate unreacted monomer acids which are then polymerised under similar conditions.

The polymerisation of fatty acids is generally approached with the objective of attaining the maximum yield of isostearic, dimeric and trimeric fatty acids. The nature of the products obtained is usually less significant than the efficiency of their formation.

We have now found that a judicious choice of process conditions leads to the formation of products, the properties of which differ substantially from those obtained by exhaustive polymerisation.

Thus, according to the invention there is provided a process for the polymerisation of an unsaturated fatty acid feedstock, wherein the fatty acid feedstock is heated to an elevated temperature in the presence of a polymerisation catalyst for a time sufficient to cause the polymerisation of less than 40% by weight of the fatty acid, and the polymerised fatty acid product is then separated from the un-polymerised fatty acid.

We have found that in the initial phase of the polymerisation process, ie. until less than 40%, preferably between 15 to 35% of the fatty acid has polymerised, the product formed is substantially easier to hydrogenate than the product of exhaustive polymerisation. Thus, according to a preferred aspect of the present invention the polymerised fatty acid product is hydrogenated, for example at a temperature of from 150 to 300°C most preferably 200-250°C at a pressure of from 5 to 150 bar (0.5 to 15 MPa), most preferably from 15 to 35 bar.

We have found that the process of the invention can be used to usefully carry out cis-trans isomerisation. Thus according to a second aspect of the invention there is provided a process for carrying out a cis-trans isomerisation of monounsaturated fatty acids, such as oleic acid, contained in a fatty acid feedstock mixture, wherein the mixture is heated to an elevated temperature in the presence of a polymerisation catalyst for a time sufficient to cause the polymerisation of less than 40% by weight of the fatty acid and the polymerised fatty acid formed is then separated from the un-polymerised fatty acid. We have found that this process may also result in an isomeric mixture product enriched in monounsaturated fatty acid.

When the feedstock contains mainly cis-monounsaturated fatty acid, the isomeric mixture product contains both cis- and trans-monounsaturated acids. An advantage of this process is that the isomeric mixture product contains less than 10%, especially less than 5% of branched fatty acids.

This aspect of the invention is particularly applicable to a fatty acid feedstock mixture which contains oleic acid, the product containing a mixture of oleic and elaidic acids, together with other isomers.

In all process aspects of the invention, the process is carried out for a time sufficient for less than 40% by weight of the fatty acid to polymerise. Typically, such as when a polymerisation temperature of more than 175°C is used, this time is up to 5 hours, such as from less than 2 hours. We prefer to use an elevated temperature of 175 to 250°C, such as from 200 - 225°C. Time is counted from the moment the temperature of the reaction mixture exceeds 60°C. A preferred heat up time is 0.25 to 3 hrs.

One characteristic of the polymerised fatty acid product which distinguishes it from the products of more exhaustive polymerisation, is the lower level of aromatic ingredients to be found therein.

Thus according to a further aspect of the invention there is provided a polymerised fatty acid product comprising primarily a dimerised fatty acid and containing less than 15%, preferably less than 10% by weight of aromatic ingredients.

A further characteristic of the polymerised fatty acid product which distinguishes it from the products of more exhaustive polymerisation, is the presence of inter-molecularly esterified fatty acids found therein. These esters are split on further reaction. The mixture of dimer fatty acids and these esters is useful in lubricants, coatings and cosmetics and they can also be used for the preparation of polyamides and polyesters.

Thus in a preferred aspect of the invention, a mixture of the polyermised fatty acid and inter-molecularly esterified fatty acids are separated from the unreacted fatty acid.

The degree of polymerisation can be determined by standard GLC (gas/liquid chromatography). After separation of the polymerised fatty acid product from the reaction mixture, there remains a number of monomeric species which may, if desired, be subjected to further polymerisation. Hydrogenation of the further polymerised product is possible such as at a pressure of 5 to 150 bar and at a temperature of 150 to 300°C and the hydrogenated further product so obtained may be characterised by a level of cyclic species therein of less than 50%, preferably less than 45% by weight.

The fatty acid feedstock useful in the process of the invention is preferably selected from natural fatty acids, typical examples of which are derived from coconut oil, cornoil, cotton seed oil, castor oil, linseed oil, peanut oil, rapeseed oil, rubber, soybean oil, safflower seed oil, sunflower, palm oil, palm kernel oil, tallow and tall oil. These sources are known to contain, in varying amounts, mono-unsaturated acids such as oleic acid, elaidic acid and palmitoleic acid and polyunsaturated acids such as linoleic and linolenic acids.

The fatty acid feedstock will usually contain some fully saturated species. These may be partly or fully removed prior to polymerisation, for example by low temperature crystallisation in methanol.

The fatty acid feedstock will usually contain some poly-unsaturated species. It is an advantage of the present invention that the un-polymerised fatty acid is lower in poly-unsaturation that the feedstock and may even be free of poly-unsaturation, while retaining the presence of mono-unsaturated fatty acids. This is thought to be due to the fact that poly-unsaturated fatty acids polymerise first in the reaction and are therefore removed as part of the polymerised fatty acid.

The polymerisation catalyst is preferably selected from clays, such as mineral clays of the type known as bentonite, hectorite, attapulgite, montmorillonite and sepiolite or synthetic alternatives thereof. The clays may be used in their natural form, as acid activated clays or as alkali metal or alkaline earth metal exchanged clays.

The polymerisation process usually takes place in the presence of a steam over-pressure. The process is preferably carried out in an autoclave, enabling a steam pressure to be generated. In addition to the fatty acids feedstock from about 1% to about 10% (based on the weight of the fatty acids) most preferably from 2% to 8% of catalyst and up to 5%, of water are included. Agitation is usually employed.

The polymerised fatty acid is separated by filtration to remove solids including the catalyst followed by distillation to remove the un-polymerised fatty acid. Cooling will generally take place before filtration. The products of the processes according to the invention may be further refined by known methods to remove contaminants.

The invention will now be illustrated in the following non-limiting examples.

## EXAMPLE 1

This example describes the polymerisation of soya fatty acids.

A 1 litre autoclave is charged with 300g fatty acid, 15.8g of montmorillonite clay, 5.25g of bleachingearth, 0.36g lithium carbonate and 6.0g of water. The autoclave is closed and the air exhausted by exchange with nitrogen. The autoclave is stirred at 500 rpm initially. Heating is begun and at about 120°C the nitrogen is released from the system - this is seen by the evolution of steam from the open valve. Stirring is then adjusted to 600 - 1500 rpm. Once the target temperature is reached, the pressure is adjusted to 7 bar overpressure. Samples are extracted from the autoclave after given periods of time. The samples were analysed in two ways, namely (i) GLC separation/analysis of methyl esters followed by mass spectrometry or (ii) HPLC separation/analysis of UV active derivative followed by mass spectometry.

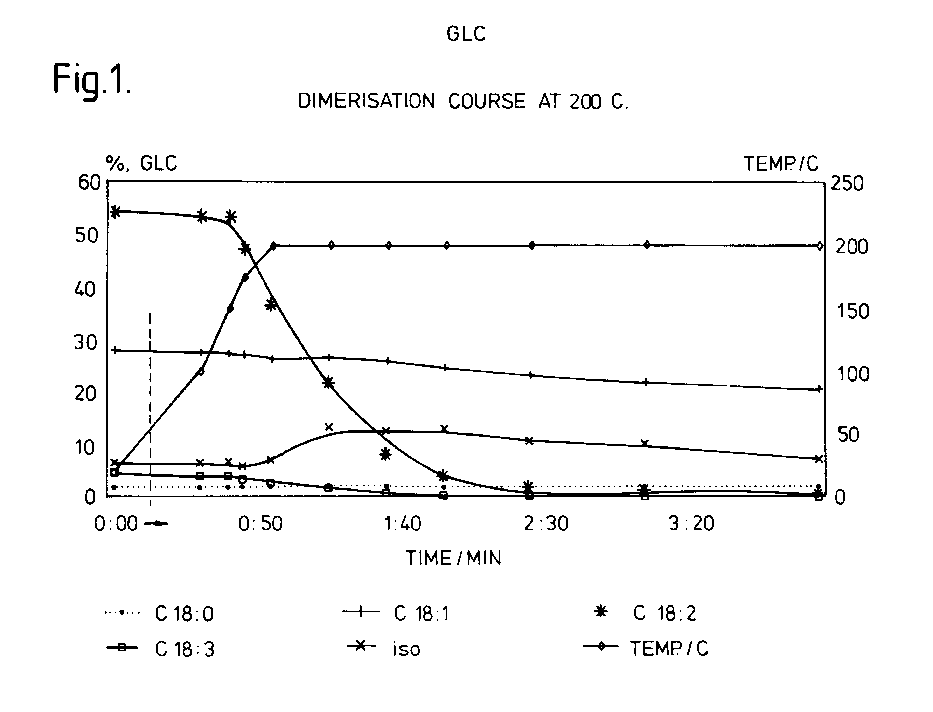

The GLC results are plotted in Figure 1, which shows the progress of the reaction with time.

From Figure 1 it will firstly be seen that the temperature inside the autoclave increased to the target of 200°C over a period of about 50 minutes, after which it remained constant. It will also be seen that the starting fatty acids contained about 55% of diunsaturated $C_{18}$ fatty acids but that this component reacted quickly and was nearly depleted after 2 hours. The level of mono-unsaturated $C_{18}$ fatty acids was about 28% in the feedstock and reacted more slowly, still being present in a significant amount at the end of the experiment.

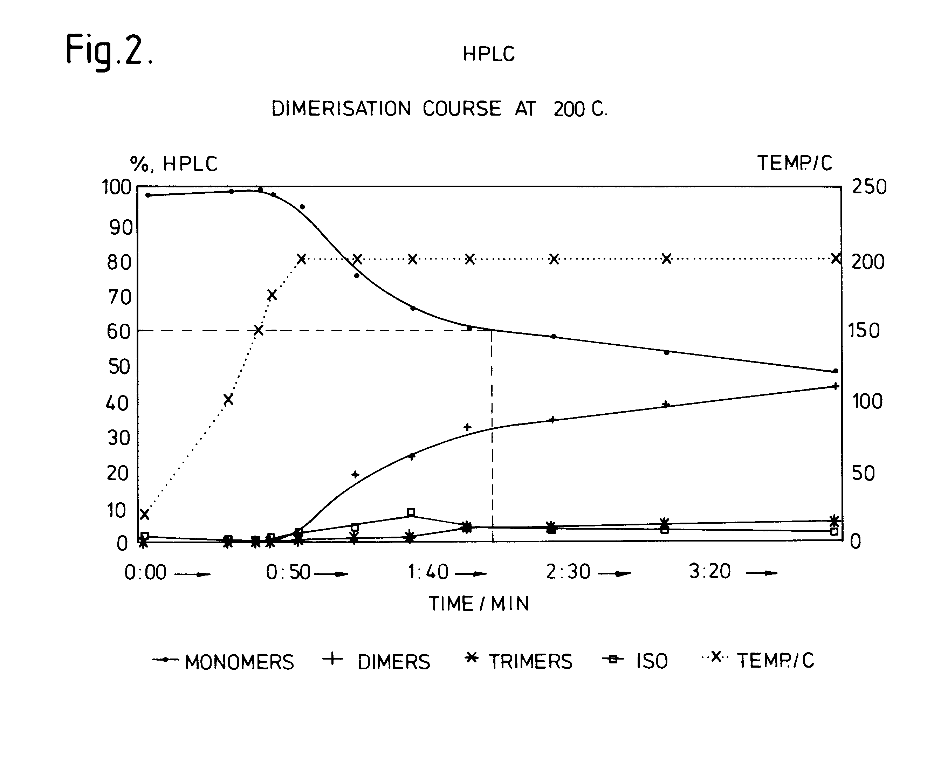

The HPLC results are plotted in Figure 2, which shows the progress of the formation of products with time.

From Figure 2 it can be seen that after about 2 hours 40% of the polymerisation reaction had taken place. From about 50 minutes until this time, the level of dimer product increased significantly.

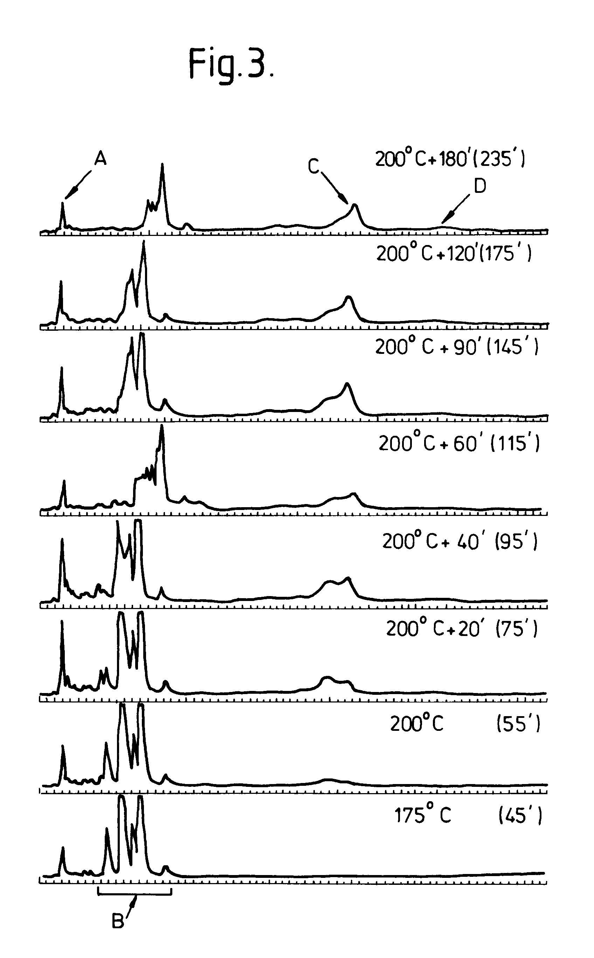

Figure 3 shows reverse phase HPLC analysis of beta-naphthacyl ester derivatives of the total dimerisation reaction mixture with UV detection at 254 nm. The chromatographic conditions are as follows:

3

```
Column:              RP-8, 7 um, 25 cm, 70°C


Eluent flow (total) 1.1 ml/min.


Gradient elution:


time/min.            solvent     solvent
                     = water     = acetonitrile
     0                 32          68
    55                  0         100
    60                  0         100
    65                 32          68


Detection            UV 254 nm
```

The traces in Figure 3 show four distinct areas. Area A is the residue from the derivatisation procedure and is irrelevant. Area B includes monomeric components. Area C includes the dimers. Area D includes the trimers.

## EXAMPLE 2

10 tonnes of mixed fatty acids of animal origin from which the saturated fatty acids had substantially been removed by low temperature crystallisation in methanol (Emersol process), were dimerised in an autoclave at 7 bar steam pressure for 2 minutes at 230°C under stirring using 3.0 weight % of montmorillonite clay as a catalyst. The composition of the feedstock is given in Table I.

After cooling to 160°C, the product was treated with 0.25 wt% of 75% phosphoric acid in water for one hour. The vacuum was subsequently removed and the product cooled and filtered. The composition of the product is given in Table II. From this table it can be concluded that the total combined dimer/trimer yield of the described (first) reaction step was 20%.

The product was dried at 100mbar and 150°C and distilled thereafter at 4mbar pressure and 265°C yielding approximatley 78% distillate and 17.5% residue. The composition of the distillate is given in Table I, from which it will be seen that substantially no polyunsatured species are present. Analytical data on the residue (first step dimer) in Table II. The large difference between the saponification value and the acid value of the first step dimer indicates the presence of a significant level of ester in the dimer fraction.

## EXAMPLE 3:

The distillate of Example 2 was reacted using 3.5 wt% of montmorillonite catalyst; 0.5 wt% Filtrol 160 bleaching earth and 0.07 wt% $Li_2CO_3$ at 7 bar steam pressure and 245°C under stirring for three hours.

The product was treated with phosphoric acid, filtered and distilled in order to separate monofunctional acids from dimerised and trimerised fatty acids at the same conditions as described in Example 2. The product composition prior to the distillation is given in Table II: From this table it can be concluded that the total combined dimer/trimer yield in this second step product was 48.5%. Analyses of monomers and oligomers after distillation are given in Tables I and II respectively. From Table II it can be concluded that only 8% of the dimer/trimer fraction consisted of aromatic species ("dimer 1"). The small difference between the saponification value and the acid value of the second step dimer indicates the substantial absence of ester in the dimer fraction.

## EXAMPLE 4:

6.5 kg of the distillation residue of Example 3 was hydrogenated in an autoclave at 25 bar hydrogen pressure and 180°C for seven hours under stirring using 91 grammes of 5 wt% palladium on carbon manufactured by Johnson-Matthhey (including 50 wt% water ie. 0.035 wt% Pd). An iodine value of $8gI_2/100g$ was reached at the end of this period. After drying and distillative "topping" of ca. 13 wt% of the product the material was purified in two passes over a two-stage short path wiped film vacuum distillation unit at the following conditions:

| Pass 1: | | Pass 2: | |
|---|---|---|---|
| Stage 1: | 0.01 mbar 240°C | 0.001 mbar 230°C | |
| Stage 2: | 0.002 mbar 295°C | 0.002 mbar 295°C | |
| Yields : | 11% top | 5% top | |
| | 68% distillate | 87% distillate | |
| | 22% residue | 8% residue | |

The distillate was collected and analysed (see Table II). From this table it can be concluded that the product contained 98.5 wt% dimerised fatty acids of which 7.5% were of aromatic nature.

## Table I: GLC-analyses of fatty acid mixtures (weight %)

| Fatty Acid Type | Feedstock | 1st step distillate | 2nd step distillate |
|---|---|---|---|
| C12 | 0.1 | 0.2 | |
| C14 | 1.7 | 2.2 | |
| "C15 gr" | 1.1 | 1.5 | |
| C16 | 4.8 | 5.8 | |
| C16:1 | 4.6 | 3.9 | |
| iso C18 | 1.9 | 5.7 | 40.9 |
| C18 | 1.9 | 2.7 | |
| C18:1 | 67.2# | 66.5* | 3.1 |
| C18:2 | 13.3 | 4.7 | |
| C18:3 | 1.4 | 1.8 | |
| C19 | 0.1 | 0.2 | |
| >C19 | 2.1 | 4.8 | |

*: ca. 35% trans C18:1; 32% cis C18:1

#: substantially all cis C18:1

**Table II:** Analysis data of dimerised fatty acids

| | 1st Step Product | 1st Step Dimer | 2nd Step Product | 2nd Step Dimer | Product Example 4 |
|---|---|---|---|---|---|
| **GLC-analysis (wt%):** | | | | | |
| monomeric acids | 79.0 | 0.2 | 49.5 | 1.1 | 0.1 |
| intermediates | 1.0 | 1.0 | 2.0 | 4.5 | 0.5 |
| dimers | 17.5 | 77.0 | 40.5 | 75.0 | 98.5 |
| trimers | 2.5 | 22.0 | 8.0 | 19.5 | 1.0 |
| | | | | | |
| **HPLC-analysis (wt%):** | | | | | |
| monomeric acids | | | | 1.5 | 0.1 |
| intermediates | | | | 3.0 | 1.0 |
| dimer 1[a] | | | | 8.0 | 7.5 |
| dimer 2+3[b] | | | | 70.5 | 87.0 |
| intermediates | | | | 4.0 | - |
| trimer | | | | 13.0 | 2.5 |

EP 0 471 566 A1

**Table II:** (Cont'd)

EP 0 471 566 A1

| | 1st Step Product | 1st Step Dimer | 2nd Step Product | 2nd Step Dimer | Product Example 4 |
|---|---|---|---|---|---|
| Other analyses: | | | | | |
| acid value (mgKOH/g) | | 156 | | 190 | 194 |
| saponification v. (") | | 186 | | 198 | |
| iodine value ($gI_2$/100g) | | | | 97 | 8 |
| dyn. viscosity | | | | | |
| (25°C) (mPa.s) | | 2030 | | 6260 | |

a: aromatic species

b: linear and cyclic (non-aromatic) species

**Claims**

1. A process for the polymerisation of an unsaturated fatty acid feedstock , wherein the fatty acid feedstock is heated to an elevated temperature in the presence of a polymerisation catalyst for a time sufficient to cause the polymerisation of less than 40% by weight of the fatty acid, and a polymerised fatty acid product is then separated from the un-polymerised fatty acid.

2. A process according to Claim 1, including the further step of hydrogenating the polymerised fatty acid product.

3. A process according to Claim 1, wherein the fatty acid feedstock is subjected to polymerisation at a temperature of at least 175°C for a time of up to 5 hours.

4. A process according to Claim 1, wherein from 15% to 35% by weight of the fatty acid is polymerised.

5. A process for carrying out a cis-trans isomerisation of monounsaturated fatty acids, such as oleic acid, contained in a fatty acid feedstock mixture, wherein the mixture is heated to an elevated temperature in the presence of a polymerisation catalyst for a time sufficient to cause the polymerisation of less than 40% by weight of the fatty acid and the polymerised fatty acid formed is then separated from the un-polymerised fatty acid.

6. A process according to Claim 5, wherein the fatty acid feedstock is subjected to polymerisation at a temperature of at least 175°C for a time of up to 5 hours.

7. A process according to Claim 5, wherein from 15% to 35% by weight of the fatty acid is polymerised.

8. A process for reducing the level of poly-unsaturated species in a fatty acid feedstock, wherein the fatty acid starting material is heated to an elevated temperature in the presence of a polymerisation catalyst for a time sufficient to cause polymerisation of less than 40% by weight of the fatty acid, and the polymerised fatty acid formed is separated to leave a monomeric fatty acid product comprising mono-unsaturated fatty acid and a reduced amount, if any, of poly-unsaturated species.

9. A process according to Claim 8, wherein the fatty acid feedstock is subjected to polymerisation at a temperature of at least 175°C for a time of up to 5 hours.

10. A process according to Claim 9, wherein from 15% to 35% by weight of the fatty acid is polymerised.

# Fig.1.

## DIMERISATION COURSE AT 200 C.

%, GLC

TEMP/C

TIME / MIN

```
·····  C 18:0        ——  C 18:1        *   C 18:2
—□—  C 18:3        —✳—  iso           —◆—  TEMP/C
```

EP 0 471 566 A1

Fig.2.

HPLC

DIMERISATION COURSE AT 200 C.

EP 0 471 566 A1

# Fig.3.

European Patent Office

# EUROPEAN SEARCH REPORT

Application Number

| | DOCUMENTS CONSIDERED TO BE RELEVANT | | EP 91307505.7 |
|---|---|---|---|

| Category | Citation of document with indication, where appropriate, of relevant passages | Relevant to claim | CLASSIFICATION OF THE APPLICATION (Int. Cl.5) |
|---|---|---|---|
| D,A | US - A - 3 632 822 <br> (N.H. CONROY) <br> * Totality * | 1,3,5, 6,8,10 | C 07 C 51/363 <br> C 07 C 57/13 |
| D,A | GB - A - 2 172 597 <br> (UNION CAMP CORP.) <br> * Totality * | 1,3,5, 6,8,10 | |
| A | DE - B - 1 134 667 <br> (EMERY IND.) <br> * Totality * | 1,3,5, 6,8,10 | |

TECHNICAL FIELDS SEARCHED (Int. Cl.5)

C 07 C 51/00
C 07 C 57/00

The present search report has been drawn up for all claims

| Place of search | Date of completion of the search | Examiner |
|---|---|---|
| VIENNA | 11-11-1991 | HOFBAUER |